(19) Europäisches Patentamt
European Patent Office
Office européen des brevets

(11) EP 0 958 825 A1

## (12) EUROPEAN PATENT APPLICATION

(43) Date of publication:
24.11.1999 Bulletin 1999/47

(51) Int. Cl.$^6$: **A61K 31/70**, A61K 31/505, A61K 31/52, A61K 31/17

(21) Application number: 98870113.2

(22) Date of filing: 18.05.1998

(84) Designated Contracting States:
AT BE CH CY DE DK ES FI FR GB GR IE IT LI LU MC NL PT SE
Designated Extension States:
AL LT LV MK RO SI

(71) Applicant:
Tiense Suikerraffinaderij N.V. (Raffinerie Tirlemontoise S.A.)
1150 Brussel (BE)

(72) Inventors:
• Taper, Henryk
  B-3012 Wilsele (BE)

• Frippiat, Anne
  B-1933 Sterrebeek (BE)
• Van Loo, Jan
  B-3000 Leuven (BE)
• Roberfroid, Marcel
  B-1180 Brussel (BE)

(74) Representative: Hermans, Johny
Tiense Suikerraffinaderij N.V.,
Patent Department,
Aandorenstraat 1
3300 Tienen (BE)

Remarks:
Claims 15-20 are deemed to be abandoned due to non-payment of the claims fees (Rule 31 (2) EPC).

(54) **Synergistic composition of a non-digestible carbohydrate and an anti-cancer drug for use in the treatment of cancer**

(57) The present invention provides (i) a novel pharmaceutical composition comprising a synergistic combination of a non-digestible fructan-type carbohydrate, i.e. a levan or an inulin (including inulin, oligofructose and mixtures thereof) and an anti-metabolic anti-cancer drug, (ii) said composition for use in the treatment of cancer in non-ruminating mammals, (iii) the use of said combination for the manufacture of a medicament for the treatment of cancer in non-ruminating mammals, and (iv) the use of said composition in a method for treatment of cancer in non-ruminating mammals.

EP 0 958 825 A1

## Description

Field of the invention

This invention relates to a pharmaceutical composition comprising a combination of a non-digestible carbohydrate and an anti-cancer drug, and to its use in the treatment of cancer in non-ruminating mammals.

Background and prior art

For various reasons, cancer has become one of the major causes of death of humans in highly developed and highly industrialised countries.

Several kinds of cancer have already been identified. Although not all mechanisms of the genesis and development of all kinds of cancer have been elucidated so far, cancer is a disease which is known to proceed in several steps, conventionally including a genesis stage, a proliferation stage and often a metastasis stage. The first stage is characterised by the genesis, out of a normal body structure, of cells of a modified genome, resulting in the formation of malignant cells. In a next stage, the malignant cells are proliferating in an uncontrolled manner and may locally invade adjacent normal body structures. In a possible later stage, termed metastasis stage, the malignant cells are spread throughout the body with invasion of various normal body structures. The invasion of normal body structures usually results in their malfunctioning and destruction, which eventually results in the death of the affected human.

On the one hand, various curative methods for the treatment of cancer have already been developed including treatment by surgery, by irradiation with X-rays, by chemotherapy or, frequently, by combinations thereof.

The method of treatment is commonly chosen taking into account *inter alia* the age and physical condition of the affected human and the kind, the location and the stage of development of the cancer. In a few cases, the treatment will be effective and able to completely cure the affected person. In most cases, however, the treatment can only delay the carcinogenesis or more or less inhibit the development of the cancer disease, and, in spite of bringing some temporary relief to the patient, the final outcome is unfortunately still fatal.

The treatment methods are continuously improving as a result of major research efforts. Invasive techniques, including surgery and irradiation, become more and more efficient as a result of technological developments, but require always more highly sophisticated equipment, making the techniques very costly and less suitable for large scale application, in the search to overcome the drawbacks of invasive treatments as well as for various other reasons, chemotherapy, in combination or not with other treatment methods, has become a major way of attack against cancer during the last two decades. A considerable advantage of anti-cancer drugs resides in the fact that they are easy to administer and current developments enable, in a steadily improving manner, target delivery of the drugs.

The anti-cancer drugs which have been developed are generally classified in one of the following groups:

1. alkylating agents, including e.g. cyclophosphamide (Endoxan[®]);
2. anti-metabolic drugs, including e.g. 5-fluorouracil;
3. anti-mitotic antibiotics, including e.g. duxorubicine;.
4. alcaloidal anti-tumor agents, e.g. vincristine sulphate (Oncovin[®]);
5. hormones and antihormones,
6. interferons, e.g. alfa-2 b interferon; and
6. various anti-tumor agents,

as disclosed for example in the Répertoire commenté des médicaments, Ed. Centre Belge d'information pharmacothérapeutique, (1987), p. 341-345.

However, most of the anti-cancer drugs have serious disadvantages and drawbacks too. They may present a high degree of toxicity for cells of normal body structures causing, for example, liver and kidney damages. They may also cause an increased sensitivity to opportunistic infections. Often they also provoke various types of discomfort for the treated person, such as, local necrosis of the body structure in which the drug is parenterally administered, and, when the drug is orally administered, nausea, vomiting, irritation of the mucoses of the digestive tract and diarrhoea. In addition to discomfort commonly caused by anti-cancer drugs, anti-metabolic anticancer drugs are known to be fairly toxic and to provoke additional discomfort, including megaloblastose, and lesions to the liver or the digestive tract such as stomatitis and buccal and gastro-intestinal ulcers. The disadvantages and drawbacks may considerably limit the use of available anti-cancer drugs. Indeed, often a curative effective dose of the drug can not be given to a patient in view of the too high toxicity of the drug to normal cells or the too high degree of discomfort caused to the patient by the drug.

On the other hand, beneficially inhibitory effects of certain dietary components on the genesis and development of cancer have been disclosed *inter alia* by Williams and Dickerson (1990); Roberfroid (1991) and Milner (1994 ). Said components, termed functional food components (Roberfroid, 1995), may have almost no nutritional and/or caloric value but interact with physiological functions in the body in a manner to improve them or to restore them more or less in case of a disfunction.

Amongst the many dietary components, non-digestible carbohydrates, commonly termed dietary fibres, have been found to present preventive effects on carcinogenesis (Nossal, 1993; Perdigon et al.,1993) and possibly inhibitory effects on the promotion of carcinogenesis (Wattenberg, 1992).

Moreover, the relation between the intake of dietary fibres and the reduction of the risk of colon cancer has been disclosed in several publications, e.g. Potter et al., (1996); Howe et al., (1992) and Reddy et al., (1992).

Dietary fibres are commonly defined as components of plant cells which are resistant to hydrolysis by the alimentary enzymes of man. Dietary fibres comprise cellulose, hemicellulose, pectin, gums, waxes, lignin and certain oligo- and polysaccharides such as fructans.

Fructans are carbohydrates which are commonly classified in levans and inulins.

Levans are D-fructans generally consisting of water soluble chains of fructose units which are mostly connected to each other by $\beta$(2-6) fructosyl-fructose linkages. Levans originate from the activity of certain bacteria and can be produced by known fermentation techniques. Levans can also be obtained by enzymatic synthesis according to conventional processes. Levans usually occur as a polydisperse mixture of polyfructose chains. These chains may be linear, but in general they are branched.

As $\beta$(2-6) fructosyl-fructose linkages are not hydrolysed by alimentary enzymes in the upper part of the digestive tract of humans, levans pass almost unaltered into the colon where they are digested by intestinal bacteria, promoting preferably the growth of beneficial bacteria.

Inulins are D-fructans consisting of water soluble chains of fructose units too, but they are composed of chains in which the fructose units are connected to each other essentially by $\beta$(2-1) fructosyl-fructose linkages. Besides, most of said inulin-type polyfructose chains end in a glucose unit. Inulin generally occurs as a polydisperse mixture of linear polyfructose chains, but the mixture may also contain, usually to a rather low extent, branched polyfructose chains.

Inulins can be represented by the general formulae $GF_n$ and $F_n$ wherein G represents a glucosyl unit, F represents a fructosyl unit, and n represents the number of fructosyl units linked to each other in the carbohydrate chain. The number of saccharide units (fructose and glucose units) in one inulin molecule, *i.e.* the values n+1 in formula $GF_n$ and n in formula $F_n$, are referred to as the degree of polymerisation, represented by (DP). Often the parameter average degree of polymerisation of the inulin, represented by $(\overline{DP})$, is used too, which is the value corresponding to the total number of saccharide units divided by the total number of inulin molecules present in a given composition, without taking into account possibly present mono and disaccharides (De Leenheer, 1996).

Inulins are synthesised by many plant species, in parts of which they may be stored as reserve carbohydrates, but inulins can also originate from bacterial activity and can be enzymatically synthesised as well. Inulin from plant origin is a polydisperse composition of polyfructose chains with a degree of polymerisation (DP) ranging from 3 to about 100, whereas inulin from bacterial origin usually has a higher (DP). The inulin profile, i.e. the distribution profile of the inulin chains according to their (DP), which to a certain extent is indicated by the average degree of polymerisation $(\overline{DP})$, depends from various factors, including the plant species, the time in the plant life cycle when the plant part is harvested, and the way the plant part is processed into inulin.

In roots of chicory and in tubers of dahlia and of Jerusalem artichoke, inulin can occur at concentrations of about 10 to 20 % on fresh weight. Inulin can be extracted easily from these plant parts, for example by extraction of the shredded plant parts with warm water. Then the crude inulin is purified and optionally fractionated to remove undesired fractions of carbohydrates. Finally, the inulin is isolated in particulate form, usually by spray drying or by directed crystallisation. These different process steps are well known in the art. At industrial scale, inulin is mainly obtained from roots of chicory.

Inulin from chicory is commercially available in various grades, for example as RAFTILINE® from ORAFTI, (Tienen, Belgium). Typical RAFTILINE® grades are, for example , ST(which has a$(\overline{DP})$ of about 10 and contains in total about 8 % by weight glucose, fructose and sucrose), LS (which has also a $(\overline{DP})$ of about 10 but which contains in total less than 1 % by weight glucose, fructose and sucrose), and HP (which has a $(\overline{DP})\geq$ 23, commonly a $(\overline{DP})$ of about 25, and is essentially free of glucose, fructose and sucrose).

Inulin-type molecules of general formulae $GF_n$ and $F_n$ having a lower degree of polymerisation (DP), usually defined as a (DP) <10, are often interchangeably termed oligofructoses, inulo-oligosaccharides and fructooligosaccharides, represented by FOS.

Unless specified otherwise, the term inulin herein is meant to comprise both inulin and oligofructose.

Oligofructose, which is thus an inulin of which the composing polyfructose chains have a (DP) < 10 , is usually obtained by partial, acidic or enzymatic hydrolysis of inulin, but can also be obtained by enzymatic synthesis from sucrose, according to techniques well-known in the art.

Several grades of oligofructose are commercially available, for example from ORAFTI (Tienen, Belgium) under the brand name RAFTILOSE®, for example: RAFTILOSE® P95 which contains about 95 % by weight oligofructose with a degree of polymerisation (DP) ranging from 2 to 7 and contains about 5 % by weight in total of glucose, fructose and sucrose.

Due to the presence of β(2-1) fructosyl-fructose linkages between the fructosyl units of the inulin-type polyfructose chains, inulin is not digested by alimentary enzymes in the upper part of the digestive tract of humans , but passes essentially unaltered into the lower part of the digestive tract to reach the colon where it is digested by intestinal bacteria, while selectively promoting the growth of certain beneficial bacteria, particularly *Bifidobacteria* (Gibson et al., 1995). This bifidogenic effect, also called prebiotic effect, is known to exert beneficial effects on various physiological functions in the body.

In view of their almost non-digestibility by alimentary enzymes of humans, fructans, levan-type fructans as well as inulin-type fructans, are considered as soluble dietary fibres.

In patent application EP 97870069.8, the use has been described of fructan-type carbohydrates, particularly chicory inulin, with an average degree of polymerisation ($\overline{DP}$) of at least 15, for the manufacture of a composition for the prevention and treatment of colon cancer in non-bovine mammals. Optionally, said composition may also comprise a physiologically active substance, a drug or pro-drug, but no particulars have been disclosed in this respect.

Furthermore, intraperitoneal injection of gamma-inulin, a specific polymorphic form of dahlia inulin, has been demonstrated to prolong the survival time of melanoma bearing mice (Cooper, 1986).

More recent studies revealed that non-digestible carbohydrates, including oligofructose and inulin, present inhibitory effects on the growth and development of intramuscularily transplanted mouse tumors (Taper et al., 1997).

Patent application EP 0 692 252 A1, discloses a composition containing inulin or oligofructose which presents a preventive effect on carcinogenesis and an inhibitory effect on the development of cancer in mammals, particularly mammary cancer.

EP 0 692 252 A1 also discloses compositions which in addition to inulin or oligofructose comprise conventional chemotherapeutic products actively destroying malignant tumor cells. In this way, the combination of the physiologically beneficial effects of non-digestible carbohydrates and of the curative effects of anti-cancer drugs are sought. Furthermore, in Example 7 (page 12 of EP 0 692 252 A1) is mentioned that to determine potential synergistic therapeutic effects, a pharmaceutical composition comprising Raftiline® (i.e. chicory inulin) and a conventional chemotherapeutic product actively destroying malignant tumor cells, is prepared and a test is described wherein doxorubicine (an anti-cancer drug of the class of the antimitotic antibiotics) was injected to mice fed oligofructose (Raftiline®) which were previously inoculated with L1210 leukaemic tumor cells.

However, EP 0 692 252 A1 is completely silent about the outcome of the test and about possible synergistic effects between a non-digestible fructan-type carbohydrate and conventional anti-cancer drugs.

<u>The problem</u>

In view of the tremendous social and economical impact of cancer on individuals as well as on society in general, huge efforts are continuously made world-wide to find new or improved products, compositions and methods for the treatment of cancer. In particular, the search is continued in order to find highly effective drugs, preferably with a low degree of toxicity to normal cells and compositions thereof, which are providing good therapeutic effects while provoking minimal discomfort to the patient.

<u>Description of the invention</u>

The present invention *inter alia* provides a solution to one or more of the problems mentioned above, by providing (i) a novel pharmaceutical composition comprising a synergistic combination of a non-digestible carbohydrate and an anti-cancer drug, (ii) said composition for use in the treatment of cancer in non-ruminating mammals, (iii) the use of said combination for the manufacture of a medicament for the treatment of cancer in non-ruminating mammals, and (iv) the use of said composition in a method for treatment of cancer in non-ruminating mammals.

By the term cancer is meant herein any kind of cancer occurring in non-ruminating mammals, irrespective of the stage of development of said cancer, thus including, in particular, the carcinogenesis stage, the proliferation stage and the metastasis stage.

The invention is based on the findings made by the inventors that the combination of certain non-digestible carbohydrates and certain anti-cancer drugs, in particular anti-metabolic anti-cancer drugs, not only results in an additive therapeutical effect vis-à-vis the genesis and development of cancer in non-ruminating mammals, but surprisingly provokes a synergistic therapeutical effect vis-à-vis said disease.

So, in one aspect the present invention relates to a novel pharmaceutical composition comprising a synergistic combination of a fructan and an anti-metabolic anti-cancer drug , which combination provokes a synergistic therapeutical effect on the genesis and development of cancer in non-ruminating mammals.

By fructan is meant herein a levan-type fructan, termed levan, as well as an inulin-type fructan, termed inulin, the latter term including inulin as well as oligofructose and mixtures thereof. Levans and inulins which are suitable in the composition according to the present invention include linear and branched chain levan, respective inulin, as well as mixtures of the respective linear and branched chain fructans.

Typically suitable inulins include chicory inulin, in particular, inulin with a $(\overline{DP})$ of about 10, such as for example RAFTILINE® ST and RAFTILINE® LS, and inulin with a $(\overline{DP}) \geq 23$, such as, for example, RAFTILINE® HP and a suitable oligofructose is, for example, RAFTILOSE® P95, all from ORAFTI (Belgium).

The term therapeutical effect used herein refers to an inhibitory effect on the genesis and development of cancer as well as to a curative effect provoking the decrease of an existing cancer and possibly the complete curing of the cancer disease.

By anti-metabolic anti-cancer drug is meant a drug selected from said class of anti-cancer drugs which embraces products which compete with the normal metabolites of nucleic acids in cell synthesis and in cell growth pathways. This class of drugs is including, inter alia, methotrexate, cytarabin, fluorouracil, mercaptopurin, thioguanine, azathioprin and hydroxycarbamide.

It is understood that the term drug used herein includes the drug per se as well as a pro-drug, i.e. a compound which in the body is transformed into the corresponding drug.

The composition according to the invention is suitable in the first place for the treatment of cancer in humans. However, it is very suitable too for the treatment of cancer in other non-ruminant mammals, such as horses, dogs and cats.

In a preferred embodiment the fructan is a chicory inulin with a $(\overline{DP})$ of about 10. In another preferred embodiment the inulin is a chicory inulin with a $(\overline{DP})$ of $\geq 23$, typically of 23 to about 25. In still another preferred embodiment, the inulin is an oligofructose, more preferably an oligofructose with a (DP) of 2 to 7.

In a further preferred embodiment, the anti-metabolic drug is 5-fluorouracil.

In a particularly preferred embodiment the fructan is chicory inulin or oligofructose and the anti-cancer drug is 5-fluorouracil.

Furthermore, a composition according to the present invention may comprise, instead of one anti-metabolic drug, a mixture of two or more antimetabolic drugs as well as a mixture of one or more anti-metabolic drugs with one or more anti-cancer drugs belonging to a different class.

The composition according to the invention can be prepared by conventional methods, comprising, for example, mixing the desired amounts of the active components, optionally in combination with one or more pharmaceutically acceptable solvents, diluents, excipients, and/or additives commonly used in galenic pharmacy.

The compositions can be presented in conventional, well known galenic forms for oral or rectal administration, or can be administered via tube feeding. The compositions for oral administration can be liquids or solids and exist, for example, as tablets, coated tablets, coated pills, capsules, granules, solutions, syrups, suspensions or emulsions. The compositions for administration via tube feeding can be solutions, syrups, suspensions or emulsions which commonly are mixed with normal food compositions for tube feeding. The compositions for rectal administration may be solids or liquids, presented in the form of suppositories, gels, solutions, suspensions or emulsions.

Suitable galenic forms also include retard release forms as well as sustained release forms which are well known in the art.

The composition according to the invention comprises at least an active dose of the fructan as well as of the anti-cancer drug or drug mixture. A dose which is effective for treatment of cancer can be formulated in the form of a single dose unit or of several partial dose units the administration of which can be spread over a certain period of time. Usually the composition according to the invention will be administered spread over several partial dose units, and spread, possibly with certain intervals, over one or more days or weeks.

The effective dose of the synergistic combination of fructan and antimetabolic anti-cancer drug will depend on various factors, including the species of non-ruminating mammal, the age and physical condition of the mammal, the kind and stage of development of the cancer, and the kind of fructan and of anti-cancer drug. The effective dose, the optimal galenic form and unit dose, and the way of administration can be determined by the skilled person following conventional methods.

A particularly interesting feature of the compositions according to the invention resides in the fact that the active ingredients of the synergistic combination, i.e. the fructan and the anti-metabolic anti-cancer drug, can be administered to the mammal (i) simultaneously and present in the same galenical formulation (constituting the pharmaceutical composition according to the present invention), or (ii) simultaneously but present in two separate galenic formulations (constituting together the pharmaceutical composition according to the present invention) and optionally via two different methods of administration, as well as (iii) separately via two separate galenic formulations (constituting together the pharmaceutical composition according to the present invention) and optionally via two different methods of administration.

It is understood that in the above mentioned methods of administration, the fructan component of the composition of the invention is administered orally, via tube feeding or rectally, whereas the anti-cancer drug can be administered orally, via tube feeding, rectally, or even parenterally.

When the fructan component and the anti-cancer drug component of the combination according to the invention are not simultaneously administered to the mammal, it is of coarse compulsory that they are brought into the body in such a manner that their physiological effects on the mammal are simultaneously present so as to enable the genesis of the synergistic effect in accordance with the present invention.

According to one preferred embodiment, both active ingredients are administered simultaneously in one pharmaceutical composition to the mammal.

In an other preferred embodiment, both active ingredients are administered simultaneously but in separate formulations. Optionally a different way of administration for each of the formulations can be used.

In a further preferred embodiment both active ingredients are administered separately in separate formulations and optionally via different methods of administration.

If the active ingredients are not administered simultaneously via the same formulation, the anti-cancer drug is preferably administered orally or parenterally, whereas the fructan is preferably administered orally or rectally. The fructan can be administered in various galenic formulations, such as for example tablets, capsules, syrups, solutions, suspensions or emulsions, but the fructan can even be administered orally in the form of a functional food or feed, i.e. an food or feed product which during its manufacture has been provided with the desired amount of fructan, such as, for example, dairy products e.g. yoghurt or cheeses; jams or marmalades; baked goods e.g. biscuits, breads or breakfast cereals; desserts e.g. puddings or ice-creams; table spreads, e.g. margarine's; drinks; meal replacers; or confectionery, e.g. gums.

In a further preferred embodiment, the same fructan as used in the composition according to the invention is already administered to the mammal some time, for example one week, before the composition of the invention is administered. Without wishing to be bound by any theory, it is supposed that as a result of the bifidogenic effect of the previously administered fructan, the intestinal flora and/or the effected physiological functions have been brought into a condition in which the synergistic effect provoked by the combination of active ingredients according to the invention can develop optimally.

In a further aspect the invention relates to a pharmaceutical composition as defined herein above for the treatment of cancer in non-ruminating mammals.

In still a further aspect, the invention relates to the use of a combination of a fructan and an anti-metabolic anti-cancer drug for the manufacture of a pharmaceutical composition as defined herein above for the treatment of cancer in non-ruminating mammals.

In still another aspect, the invention relates to a method for the treatment of cancer in non-ruminating mammals, comprising administering to said mammal in need for such treatment an effective dose of the pharmaceutical composition comprising a synergistic combination of a fructan and an anti-metabolic and-cancer drug as defined above, said active ingredients, i.e. the fructan and the anti-cancer drug, being administered either simultaneously in the same or separate formulations, or separately in separate formulations, as described herein before.

Examples

In support of the present invention, the following illustrative data are given regarding experiments wherein the therapeutical effects on cancer in mice of a composition according to the present invention are compared with the therapeutical effects of a composition containing a combination of a fructan with an anti-cancer drug which belongs to a different class.

To investigate whether dietary treatment with inulin ( inulin or oligofructose [FOS] ) would be able to potentiate the therapeutic effects of anti-cancer drugs commonly utilised in human cancer treatment,, representatives of 4 principal classes of anti-cancer drugs have been chosen and compared in the following test.

The drugs were: endoxan (belonging to the class of alkylating agents), adriblastina (adriamycin) ( belonging to the class of antimitotic antibiotics), 5-fluorouracil (belonging to the class of anti-metabolic agents) and oncovin (belonging to the class of antineoplastic alkaloids.

Viable, neoplastic cells ($10^6$) from the ascitic form of a transplantable mouse liver tumor (TLT) (Taper, 1966 and Cappurino, 1966) were intraperitoneally transplanted into 12 per group young adult NMRI male mice weighing about 30 g, supplied by "Animalerie Facultaire" UCL, Brussels. Mice of the experimental group were fed with 15 % FOS or inulin containing basal diet starting 7 days before tumor transplantation up to the end of the experiment. Control animals received basal diet for experimental animals AO4 furnished by UAR, Villemoisson sur Orge, France, and water ad libitum.

A single, subtherapeutic dose of cytotoxic drugs was intraperitoneally injected 48 hours after intraperitoneal TLT tumor transplantation at following doses: endoxan 80 mg/kg; adriamycin 0.1 mg/kg; oncovin 0.5 mg/kg and 5-fluorouracil 40 mg/kg.

The criteria for the evaluation of results were the mortality rates expressed by mean survival time (MST) and the percentage of increase of life span (ILS) in experimental groups compared with the controls calculated according to the NCJ instructions (Geron et al., 1972). The results of each experiment were confirmed by another experiment performed at another time and were cumulatively calculated as presented in Table 1. The results concerning the survival rates of the ascitic form of TLV tumor bearing mice observed in 2 separately performed experiments on each type of anti-cancer drug (12 mice per group) are cumulatively presented in Table 1 below.

In all the experiments a chemotherapy potentiating effect has been observed for dietary FOS or inulin. Obviously this therapeutic effect was somewhat different for the various drugs. Only for 5-fluorouracil treatment a synergistic therapeutic effect has bee observed. For treatment with oncovin, endoxan and adriamycin, the therapeutic effects had only additive character. There is no significant difference observed between treatment with inulin or FOS.

All results of supplementary dietary FOS treatment were statistically very highly significant ($p < 0.001$). There were no negative results in any of the separately performed experiments. 'Furthermore, no gastro-intestinal troubles were observed in all animals after the direct introduction of 15 % FOS or inulin in the diet. There was not any sign of the increased drug toxicity after dietary introduction of both fructans (e.g. there was not any difference in body weights between only with cytotoxic drug treated mice and those with supplementary fructans).

Furthermore, it should be noted that in the experiments unfavourable therapeutic conditions were deliberately utilised by the administration of a single, sub-therapeutic dose of cytotoxic drug 48 hours after the intraperitoneal transplantation of a very aggressive and rapidly growing ascitic form of mouse tumor.

TABLE 1

| Therapeutic effects of FOS or inulin associated to a single dose of different cytotoxic drugs administered to ascitic TLT tumor bearing mice | | | |
|---|---|---|---|
| Treatment | ILS (%) | Effect of combined treatment(*) | Statistical significance |
| 5-Fluorouracil | 18.75 | | |
| FOS | 12.5 | | |
| 5-Fluorouracil + FOS | 40.6 | Synergetic | $p < 0.001$ |
| Adriamycin | 14.7 | | |
| FOS | 5.9 | | |
| Adriamycin + FOS | 17.6 | Additive (*) | $p < 0.001$ |
| Endoxan | 0 | | |
| FOS | 25.0 | | |
| Endoxan + FOS | 25.0 | Additive (*) | $p < 0.001$ |
| Oncovin | 33.33 | | |
| FOS | 13.33 | | |
| Inulin | 10.0 | | |
| Oncovin + FOS | 46.66 | Additive (*) | $p < 0.001$ |
| Oncovin + inulin | 43.33 | Additive (*) | $p < 0.01$ |
| Each of the differently treated group of 12 mice had its individual, untreated, control group to which it was compared in the calculation of the increase of life span (ILS). The cumulatively presented results are based on 2 experiments independently performed at different time. Lagrank test was utilized for statistical analysis of the results. | | | |

(*) Comparative

From the comparative data presented in Table 1 clearly follows that the composition according to the present invention comprising a combination of a fructan and an anti-metabolic anti-cancer drug has a synergistic therapeutical effect on cancer in mice, whereas for the compositions containing a fructan and an anti-cancer drug of an other class only a merely additional effect is observed. Besides, the compositions of the invention provoked no disadvantageous effects on the digestive tract and were not potentiating the toxicity of the anti-cancer drug.

As a result of said synergistic effect the compositions of the invention present considerable advantages over the conventional compositions. For example, compared to a conventional or comparative compositions containing the same concentration of active components, the therapeutical effect of a composition of the invention is significantly increased which may lead to a considerable improvement of the bodily condition of the treated mammal and which may enable to better control the disease and even enable to have restored to a more or less extent the normal physiological functions and affected body structures. On the other hand, said synergistic effect can enable the use of certain anti-cancer drugs which are fairly toxic or provoke disadvantageous side effects in compositions according to the invention in reduced concentrations while still maintaining the same or a desirable level of therapeutical effect. Said synergistic effect makes it even possible to use certain anti-cancer drugs which, at the concentration they have to be used in conventional composition to obtain a desired therapeutical effect, are too toxic or provoke such undesirable side effects that they can not be supported by the treated mammal.

In addition, the treated mammal also enjoys the known beneficial effects resulting from the bifidogenic effect of the fructan which is present in the composition of the invention.

Consequently, it can be concluded that the composition and method according to the invention present a considerable improvement in the fight against cancer in non-ruminating mammals.

REFERENCES

Williams, C.M., Dickerson, J.W., Nutr. Res. Rev., 3, 45-100, (1990)
Roberfroid, M.B., Mutation Res., 259, 351-362, (1991)
Milner, J.A., Functional Foods, pp. 39-70, Chapman and Hall, New York, London (1994)
Roberfroid, M.B., World of ingredients, 3, 42-44 (1995)
Nossal, G.J.V., Sci. Amer., 269, 21-30 (1993)
Perdigon, G. et al., Int. J. Immunother., 9, 29-52 (1993)
Wattenberg, L., Cancer; Res., 52, 2085s-2091s, (1992)
Potter, J. et al., Principles of Chemoprevention, IARC Scientific Publication N° 139, 61-90, (1996);
Howe, G.R. et al., J. Natl. Cancer Inst., 84, 1887-1896, (1992);
Reddy, B.S. et al., Gastroenterol., 102, 1475-1482, (1992).
De Leenheer, L., Carbohydrates as Organic Raw Materials, Vol. III, p.67-92, (1996)
Gibson, G.R. et al., Gastroenterology,108, 975-982, (1995) and J. Nutr., 125, 1401-1412, (1995)
Cooper, P.D. et al., Mol.Immunol., 23, 903-908, (1986)
Taper, H.S. et al., Int. J. Cancer, 71, 1109-1112 (1997).
Taper, H.S. et al., Cancer Res., 26, 193-198, (1966)
Cappurino, J.G. et al., Cancer Res., 26, 689-694, (1966)
Geron, R.J. et al., Cancer Chemother. Reports, 3, 1-103, (1972).

**Claims**

1.  Pharmaceutical composition comprising a synergistic combination of a fructan and an anti-metabolic anti-cancer drug.

2.  Pharmaceutical composition according to claim 1 wherein the fructan is levan.

3.  Pharmaceutical composition according to claim 1 wherein the fructan is inulin or oligofructose or a mixture thereof.

4.  Pharmaceutical composition according to any one of claims 1 to 3 wherein the anti-cancer drug is selected from the group consisting of methotrexate, cytarabin, fluorouracil, mercaptopurin, thioguanin, azathioprin and hydroxycarbamide.

5.  Pharmaceutical composition according to claim 4 wherein the fructan is inulin, oligofructose or a mixture thereof, and the anti-cancer drug is 5-fluorouracil.

6.  Pharmaceutical composition according to any one of claims 1 to 5 which additionally to the said anti-metabolic anti-cancer drug contains one or more anti-cancer drugs belonging to the class of anti-metabolic anticancer drugs and/or to another class of anti-cancer drugs.

7.  Pharmaceutical composition according to any one of claims 1 to 6 in which the fructan and the anti-metabolic anti-cancer drug which constitute the synergistic combination are simultaneously present in the same galenic formulation.

8. Pharmaceutical composition according to any one of claims 1 to 6 in which the fructan and the anti-metabolic anti-cancer drug which constitute the synergistic combination are present in separate formulations which together form the pharmaceutical composition.

9. Pharmaceutical composition according to any one of claims 1 to 8 wherein the single galenic formulation or the separate galenic formulations forming the pharmaceutical composition is suitable for oral administration, rectal administration, or for tube feeding.

10. Pharmaceutical composition according to claim 8 in which the fructan is present in a functional food or feed.

11. Pharmaceutical composition according to claim 8 in which the anti-cancer drug is present in a formulation which is suitable for parenteral administration.

12. Pharmaceutical composition according to any one of claims 1 to 11 for use as a medicament for the treatment of cancer in non-ruminating mammals.

13. Pharmaceutical composition according to any one of claims 1 to 11 for use as a medicament for the treatment of cancer in humans.

14. Use of a combination of a fructan and an anti-metabolic anti-cancer drug for the manufacture of a pharmaceutical composition as defined in any one of claims 1 to 11 for the treatment of cancer in a non-ruminating mammal.

15. Method for the treatment of cancer in a non-ruminating mammal comprising administering to said mammal in need of such treatment an effective amount of a pharmaceutical composition as defined in any one of claims 1 to 11.

16. Method according to claim 15 wherein the fructan and the anti-metabolic anti-cancer drug of the synergistic combination of the composition are present in the same galenic formulation constituting the pharmaceutical composition.

17. Method according to claim 15 wherein the fructan and the anti-metabolic anti-cancer drug of the synergistic combination of the pharmaceutical composition are present in separate galenic formulations constituting together the pharmaceutical composition.

18. Method according to claim 17 wherein the separate galenic formulations are administered simultaneously or separately.

19. Method according to claim 17 wherein the separate galenic formulations are administered via different methods of administration, the fructan containing formulation orally, via tube feeding or rectally, and the anti-cancer drug containing formulation orally, via tube feeding, rectally or parenterally.

20. Method according to claim 19 wherein the separate galenic formulation containing the fructan is a functional food or feed.

EP 0 958 825 A1

| European Patent Office | **PARTIAL EUROPEAN SEARCH REPORT** which under Rule 45 of the European Patent Convention shall be considered, for the purposes of subsequent proceedings, as the European search report | **Application Number** EP 98 87 0113 |
|---|---|---|

## DOCUMENTS CONSIDERED TO BE RELEVANT

| Category | Citation of document with indication, where appropriate, of relevant passages | Relevant to claim | CLASSIFICATION OF THE APPLICATION (Int.Cl.6) |
|---|---|---|---|
| X,D | EP 0 692 252 A (RAFFINERIE TIRLEMONTOISE SA) 17 January 1996 * page 3, line 5-29 * | 1-20 | A61K31/70 A61K31/505 A61K31/52 A61K31/17 |
| X | LEIBOVICI J. ET AL: "Combined effect of levan and cytotoxic agents on the growth of experimental tumours in mice" BR. J. EXP. PATHOL., 1983, 64/3 (239-244), XP002083711 ENGLAND * abstract * | 1,2,4, 7-20 | |

TECHNICAL FIELDS SEARCHED (Int.Cl.6)

A61K

### INCOMPLETE SEARCH

The Search Division considers that the present application, or one or more of its claims, does/do not comply with the EPC to such an extent that a meaningful search into the state of the art cannot be carried out, or can only be carried out partially, for these claims.

Claims searched completely :

Claims searched incompletely :

Claims not searched :

Reason for the limitation of the search:

Although claims 15-20 are directed to a method of treatment of the human/animal body (Article 52(4) EPC), the search has been carried out and based on the alleged effects of the compound/composition.

| Place of search | Date of completion of the search | Examiner |
|---|---|---|
| THE HAGUE | 9 November 1998 | Leherte, C |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C07)

10